(19) Europäisches Patentamt
European Patent Office
Office européen des brevets

(11) Veröffentlichungsnummer: **0 272 594**
**A1**

(12) # EUROPÄISCHE PATENTANMELDUNG

(21) Anmeldenummer: **87118639.1**

(22) Anmeldetag: **16.12.87**

(51) Int. Cl.[4]: **C07D 471/04** , C07D 487/04 , C07D 498/04 , C07D 513/04 , A01N 43/90 , //(C07D471/04, 235:00,221:00)

Claims for the following Contracting State: ES.

(30) Priorität: **20.12.86 DE 3643748**

(43) Veröffentlichungstag der Anmeldung:
**29.06.88 Patentblatt 88/26**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Liebl, Rainer, Dr.**
**An der Weinleite 5b**
**D-8901 Todtenweis(DE)**
Erfinder: **Frey, Michael, Dr.**
**Meraner Strasse 26a**
**D-8902 Neusäss(DE)**
Erfinder: **Mildenberger, Hilmar, Dr.**
**Fasanenstrasse 24**
**D-6233 Kelkheim (Taunus)(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**

(54) **Bicyclische Imide, Verfahren zu ihrer Herstellung sowie ihre Verwendung im Pflanzenschutz.**

(57) Verbindungen der Formel I oder deren Salze

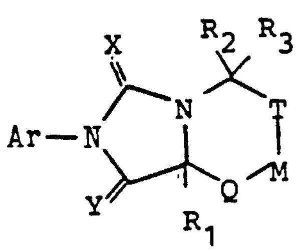

(I),

worin Ar = einen gegebenenfalls substituierten Phenyl-, Naphthyl-, Pyridyl-, Chinolinyl-oder Isochinolinyl-Rest, $R_1$ = H, Alkyl oder (subst.) Phenyl; $R_2$, $R_3$ = H, (subst.) Alkyl, (subst.) Phenyl, (subst.) Benzyl, Alkoxy, (subst.) Alkoxycarbonyl, Carboxy, $-CONR_8R_9$, $-CONR_1-NR_{10}R_{11}$, $-C(R_1)=N-NR_{10}R_{11}$, einen Rest

EP 0 272 594 A1

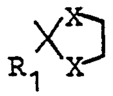

. M = $>CR_2R_3$, S, SO, $SO_2$, O oder $NR_7$; Q = $>CR_2R_3$, S oder O; T = $>CR_2R_3$, S, SO, $SO_2$ oder O; X = O oder S; Y = O, S oder NH; Z = O, S oder $NR_6$; m = 1, 2, 3; n = 1 oder 2 bedeuten, mit der Maßgabe, daß wenn Y = O; $R_1$, $R_2$, $R_3$ = H und Q,T = $CH_2$ bedeuten, M nicht $CH_2$, S, SO oder $SO_2$ sein darf, besitzen vorteilhafte herbizide Eigenschaften und eignen sich insbesondere für den Einsatz in der Landwirtschaft.

## Bicyclische Imide, Verfahren zu ihrer Herstellung sowie ihre Verwendung im Pflanzenschutz

Bicyclische Imide mit herbizider Wirksamkeit sind in EP-A 70 389, EP-A 104 532 und US-PS 4 179 276 beschrieben.

Es wurden nun überraschenderweise neue bicyclische Imide gefunden, die eine deutlich bessere herbizide Wirksamkeit bei hervorragender Selektivität aufweisen.

Gegenstand der vorliegenden Erfindung sind daher Verbindungen der Formel I oder deren Salze

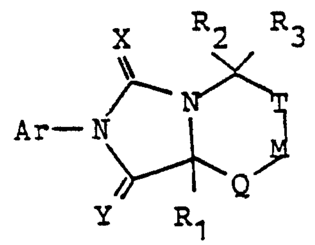 (I),

worin

Ar = Phenyl, Naphthyl, Pyridyl, Chinolinyl oder Isochinolinyl bedeuten, wobei diese Reste ein-bis vierfach, vorzugsweise ein-bis dreifach, durch gleiche oder verschiedene Reste der Gruppe Halogen, Hydroxy, $C_1$-$C_4$)Alkyl, Halo($C_1$-$C_4$)alkyl, ($C_1$-$C_4$)Alkoxy, ($C_3$-$C_6$)-Cycloalkoxy, ($C_3$-$C_6$)Alkenyloxy, ($C_3$-$C_6$)Alkinyloxy, ($C_1$-$C_4$)-Alkoxy-($C_1$-$C_4$)alkoxy, Halo($C_1$-$C_4$)alkoxy, Halo($C_3$-$C_6$)alkenyloxy, Halo($C_3$-$C_6$)alkinyloxy, ($C_1$-$C_4$)Alkylthio, ($C_1$-$C_4$)Alkylsulfinyl, ($C_1$-$C_4$)Alkylsulfonyl, $NO_2$, -CN, -$NHR_1$, Cyano($C_1$-$C_4$)alkyl, Phenoxy, Phenoxy($C_1$-$C_4$)alkyl, Phenyl($C_1$-$C_4$)alkoxy, Phenylthio, Phenylsulfinyl, Phenylsulfonyl, wobei die sechs letztgenannten Reste im Phenylring bis zu dreifach durch Halogen, ($C_1$-$C_4$)Alkyl, ($C_1$-$C_4$)Alkoxy, ($C_1$-$C_4$) Alkoxycarbonyl, -CN oder $NO_2$ substituiert sein können, oder ferner durch einen Rest der Formeln

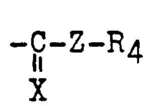 , 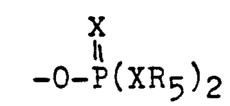 oder 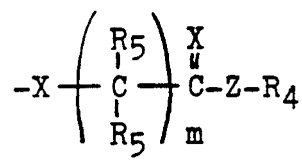

substituiert sein können;

$R_1$ Wasserstoff, ($C_1$-$C_4$)Alkyl oder Phenyl, das bis zu zweifach durch Halogen, ($C_1$-$C_4$)Alkyl, ($C_1$-$C_4$)Alkoxy, $NO_2$, CN oder ($C_1$-$C_4$)Alkoxycarbonyl substituiert sein kann,

$R_2$,$R_3$ unabhängig voneinander Wasserstoff, ($C_1$-$C_4$)Alkyl, das durch Cyano, Hydroxy oder ($C_1$-$C_4$)Alkoxy substituiert sein kann, Phenyl oder Benzyl, die beide im Phenylring jeweils vorzugsweise bis zu 2-fach durch Halogen, ($C_1$-$C_4$)Alkyl, ($C_1$-$C_4$)Alkoxy, $NO_2$ oder -CN substituiert sein können;

($C_1$-$C_4$)Alkoxy, ($C_1$-$C_4$)Alkoxycarbonyl, Halogen($C_1$-$C_4$)alkoxycarbonyl, ($C_1$-$C_4$)-Alkoxy($C_1$-$C_4$)alkoxycarbonyl, ( $C_1$-$C_4$)Alkoxycarbonyl($C_1$-$C_4$)alkoxycarbonyl, Carboxy, oder einen Rest der Formeln

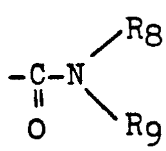 , 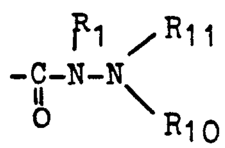 , 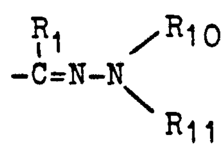 oder

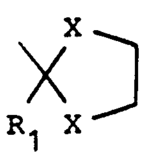

$R_4$ Wasserstoff, ($C_1$-$C_4$)Alkyl, das bis zu sechsfach durch Halogen und/oder bis zu zweifach durch ($C_1$-$C_4$)-Alkoxy, ($C_1$-$C_4$)Alkoxy($C_1$-$C_4$)alkoxy, ($C_1$-$C_4$)Alkoxycarbonyl, ($C_1$-$C_4$)Alkylthio, ($C_1$-$C_4$)Alkylsulfinyl, ($C_1$-$C_4$)-Alkylsulfonyl, ($C_1$-$C_4$)Alkylamino, -CN, Furyl, Tetrahydrofuryl, Benzofuryl, Phenyl, Phenoxy, Benzyloxy, wobei bei den sechs letztgenannten Resten jeweils der Phenylring oder der Heteroaromat bis zu dreifach durch Halogen, ($C_1$-$C_4$)Alkyl oder ($C_1$-$C_4$)Alkoxy substituiert sein kann, ($C_3$-$C_6$)Cycloalkyl, ($C_3$-$C_6$)Alkenyl, Cyclohexe-

nyl, ($C_3$-$C_6$)Alkinyl, Phenyl, das bis zu dreifach substituiert sein kann durch Halogen, ($C_1$-$C_4$)Alkyl, ($C_1$-$C_4$)-Alkoxy oder ($C_1$-$C_4$)Alkoxycarbonyl; oder einen Rest der Formel

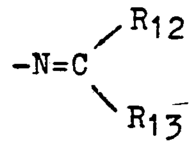

wobei der letztgenannte Rest für Z = S ausgenommen ist,

$R_5$ Wasserstoff oder ($C_1$-$C_4$)Alkyl,

$R_6$ Wasserstoff, ($C_1$-$C_4$)Alkyl oder zusammen mit $R_4$ und dem diese Reste verbindenden Stickstoffatom einen 5-bis 7-gliedrigen Heterocyclus, der als Ringglieder ein oder zwei Reste der Gruppe -O-, -S-, -$NR_5$-enthalten kann und bis zu 3-fach durch ($C_1$-$C_4$)Alkyl substituiert sein kann;

$R_7$ Wasserstoff, ($C_1$-$C_4$)Alkyl, Phenyl oder Benzyl, wobei der Phenylring jeweils bis zu dreifach substituiert sein kann durch Halogen, ($C_1$-$C_4$)Alkyl, ($C_1$-$C_4$)Alkoxy, ($C_1$-$C_4$)Alkoxycarbonyl, -$NO_2$, $CF_3$, -CN oder einen Rest der Formeln

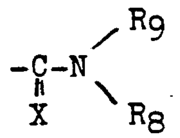 , 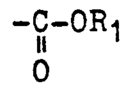 , 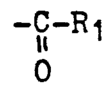 , 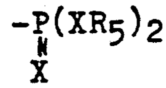 oder

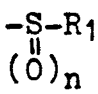

$R_8$,$R_9$ unabhängig voneinander Wasserstoff oder ($C_1$-$C_4$)Alkyl oder beide Reste $R_8$, $R_9$ zusammen mit den sie verbindenden Stickstoffatom einen 5-bis 7-gliedrigen Heterocyclus, der als Ringglieder ein oder zwei Reste der Gruppe -O-, -S0, -$NR_5$-enthalten kann und der bis zu dreifach durch ($C_1$-$C_4$)Alkyl, Hydroxy, ($C_1$-$C_4$)-Alkoxy, Phenyl oder Benzyl, die beide im Phenylring, vorzugsweise bis zu dreifach, durch ($C_1$-$C_4$)Alkyl, ($C_1$-$C_4$)Alkoxy, Halogen oder ($C_1$-$C_4$)Alkoxycarbonyl substituiert sein können, substituiert sein kann,

$R_{10}$,$R_{11}$ unabhängig voneinander Wasserstoff, ($C_1$-$C_4$)Alkyl, Phenyl oder Benzyl, die beide jeweils im Phenylring, vorzugsweise bis zu dreifach, substituiert sein können durch Halogen, ($C_1$-$C_4$)Alkyl, ($C_1$-$C_4$)-Alkoxy, $NO_2$, -CN, $CF_3$ oder ($C_1$-$C_4$)Alkoxycarbonyl, oder $R_{10}$ und $R_{11}$ zusammen den Rest

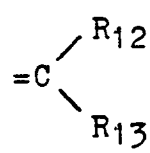 ,

$R_{12}$,$R_{13}$ unabhängig voneinander, ($C_1$-$C_4$)Alkyl, ($C_1$-$C_4$)Alkoxycarbonyl, Phenyl oder Benzyl, die beide im Phenylring, vorzugsweise bis zu dreifach, substituiert sein können durch Halogen, ($C_1$-$C_4$)Alkyl, ($C_1$-$C_4$)-Alkoxy, ($C_1$-$C_4$)Alkoxycarbonyl, $CF_3$, -CN oder $NO_2$,

M 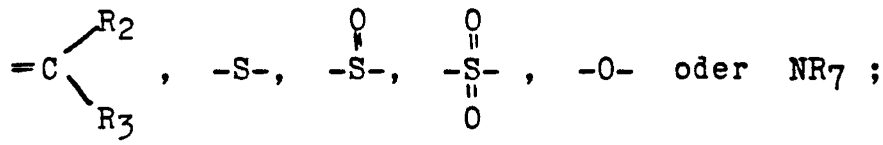

Q 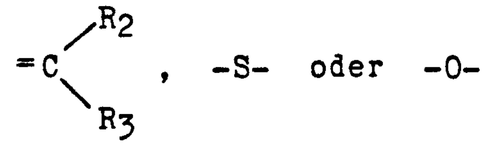

T 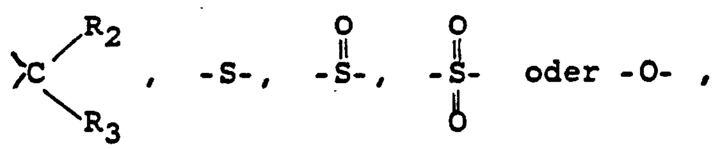

X = O oder S
Y = O, S oder NH,
Z = O, S oder $NR_6$,
m = 1, 2, 3 und
n = 1 oder 2 bedeuten,
mit der Maßgabe, daß, wenn Y = O, $R_1$, $R_2$, $R_3$ = H und Q, T = $CH_2$ bedeuten, M nicht $CH_2$, S, SO oder $SO_2$ sein darf.

Die Verbindungen der Formel I können als reine Stereoisomere oder deren Gemische vorliegen. Alle diese Isomerenformen werden von der Erfindung erfaßt.

Die Salzbildung bei den Verbindungen der Formel I kann erfolgen, wenn $R_2$ oder $R_3$ = Carboxy oder wenn $ZR_4$ = OH oder SH bedeutet. Als Salze kommen allgemein solche in Betracht, die in der Landwirtschaft einsetzbar sind. Hierzu zählen beispielsweise die Alkali-, Erdalkali-Salze insbesondere Na-, K-, Mg-, Ca-Salze, oder die Salze mit Ammonium, das ein-bis vierfach durch organische Reste, insbesondere Alkyl oder Hydroxyalkyl-Reste substituiert sein kann.

In der Definition von Formel I enthält Haloalkyl, Haloalkoxy, Haloalkenyloxy oder Haloalkinyloxy ein oder mehrere Halogenatome vorwiegend ein bis sechs F-, Cl-oder Br-Atome. Hierzu zählen beispielsweise die Reste $-CF_3$, $-C_2F_5$, $-CH_2CF_3$, $-CF_2Cl$, $-CF_2CHF_2$, $-CF_2CHFCl$, $-CF_2-CH_2-CF_3$, $-CF_2-CHFBr$, $-OCF_3$, $-OCF_2-CHF_2$, $-OCF_2-CHFCl$, $-OCF_2-CHCl_2$, $-OCF_2-CHFBr$, $-OCF_2-CHF-CF_3$

Als heterocyclische Reste für die Gruppierung $R_6$-N-$R_4$ oder $R_8$-N-$R_9$ kommen insbesondere infrage Piperidin, Pyrrolidin, Morpholin oder 2,6-Dimethylmorpholin.

Bevorzugt von den Verbindungen der Formel I sind solche Verbindungen bei denen
Ar = Phenyl, das bis zu dreifach substituiert sein kann durch Fluor, Chlor oder Brom, $(C_1-C_4)$Alkyl, $(C_1-C_4)$-Alkoxy, $(C_3-C_4)$Alkenyloxy, $(C_3-C_4)$Alkinyloxy, Halo$(C_1-C_4)$alkyl, Halo$(C_1-C_4)$alkoxy, $-NHR_1$, $(C_1-C_4)$Alkylthio, $(C_1-C_4)$Alkylsulfonyl, $NO_2$ oder einen Rest der Formeln

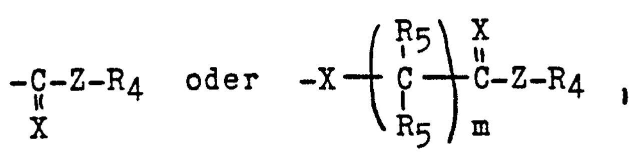

$R_1$ = Wasserstoff, $(C_1-C_4)$Alkyl, $(C_1-C_4)$Alkoxycarbonyl
$R_2$,$R_3$ = unabhängig voneinander Wasserstoff, $(C_1-C_4)$Alkyl, $(C_1-C_4)$Alkoxycarbonyl, $(C_1-C_4)$Alkoxy$(C_1-C_4)$-alkoxycarbonyl, Halogen$(C_1-C_4)$alkoxycarbonyl, $(C_1-C_4)$Alkoxycarbonyl$(C_1-C_4)$alkoxycarbonyl, -CN,Carboxy,

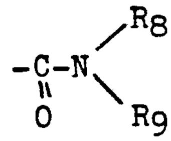

$R_4$ = $(C_1\text{-}C_4)$Alkyl, Halogen$(C_1\text{-}C_4)$alkyl, $(C_1\text{-}C_4)$Alkoxyalkyl, $(C_1\text{-}C_4)$Alkoxycarbonyl$(C_1\text{-}C_4)$alkyl;
$R_5$ = H oder $(C_1\text{-}C_4)$Alkyl,
$R_7$ = Wassestoff, $(C_1\text{-}C_4)$Alkyl,

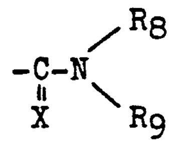 ;

Phenyl oder Benzyl, die beide bis zu zweifach durch $(C_1\text{-}C_4)$Alkyl, $(C_1\text{-}C_4)$Alkoxy, Halogen, $NO_2$, oder $-CF_3$ substituiert sein können,
$R_8$,$R_9$ = Wasserstoff, $(C_1\text{-}C_4)$Alkyl, zusammen mit den sie verbindenden N-Atom einem 6-gliedrigen gesättigten Heterocyclus

M = 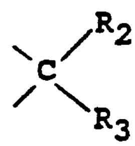 , -O- oder $-NR_7-$

Q = 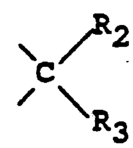

T = 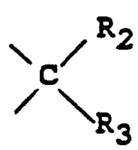 oder -S-

X = O oder S,
Y = O oder NH,
Z = O oder S und
m = 1 bedeuten.

Besonders bevorzugte Verbindungen der Formel I sind solche Verbindungen bei denen
Ar = Phenyl, das bis zu dreifach substituiert sein kann durch Fluor, Chlor, Brom, $(C_1\text{-}C_4)$Alkyl, $(C_1\text{-}C_4)$Alkoxy, Halo$(C_1\text{-}C_4)$alkoxy, $(C_3\text{-}C_4)$Alkenyloxy, $(C_3\text{-}C_4)$Alkinyloxy oder $(C_1\text{-}C_4)$Alkylthio,
$R_1$ = Wasserstoff oder $(C_1\text{-}C_4)$Alkyl,
$R_2$, $R_3$ = unabhängig voneinander Wasserstoff oder $(C_1\text{-}C_4)$Alkyl,
M = > $CR_2R_3$ oder Sauerstoff,
Q, T = >$CR_2R_3$ und
X, Y = Sauerstoff bedeuten.

Ein weiterer Gegenstand der Erfindung sind Verfahren zur Herstellung von Verbindungen der Formel (I) oder deren Salze, dadurch gekennzeichnet, daß man

a) für Y = O, S eine Verbindung der Formel II mit einer Verbindung der Formel III,

Ar-N=C=X

(II)

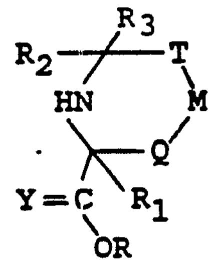

(III)

worin R = H oder ($C_1$-$C_4$)Alkyl bedeutet, oder

b) eine Verbindung der Formel II mit einem Amin der Formel IV umsetzt

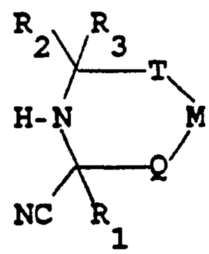

IV

oder

c) für Y = O eine unter b) erhaltene Verbindung der Formel I hydrolysiert und die erhaltenen Verbindungen gegebenenfalls in ihre Salze überführt.

Bei der Verfahrensvariante a) erfolgt die Umsetzung für R = Alkyl in einem inerten organische Lösungsmittel beispielsweise einem aromatischen Lösungsmittel wie Toluol, Chlorbenzol, einem halogenierten Kohlenwasserstoff wie Chloroform, einem Ether wie Di-isopropylether oder in Dimethylformamid, gegebenenfalls unter Basenkatalyse bei Temperaturen von 20 bis 120°C, vorzugsweise 60 bis 100°C. Als Basen werden vorzugsweise organische Basen beispielsweise organische Amine wie Triethylamin oder auch Pyridin eingesetzt.

Die Umsetzung für R = Wasserstoff kann auch in Wasser als Lösungsmittel durchgeführt werden oder vorzugsweise im 2-Phasen-System Wasser/organisches Lösungsmittel. Besonders bevorzugt wird dabei die Arbeitsweise, bei der die Verbindung der Formel III mit einer anorganischen Base, beispielsweise einem Alkali-oder Erdalkalihydroxid, -carbonat oder -hydrogencarbonat wie Natriumhydroxyd oder auch Kaliumcarbonat, oder einer organischen Base beispielsweise einem organischen Amin wie Triethylamin in das Anion überführt wird.

Zu der Lösung des Anions in Wasser wird dann das Isocyanat bzw. Isothiocyanat der Formel II, gelöst in einem inerten organischen Lösungsmittel wie z.B. Toluol, Chlorbenzol, Chloroform unter kräftigem Rühren zugetropft.

Die wäßrige Phase wird dann mit einer Säure, vorzugsweise mit einer Mineralsäure wie Salzsäure oder Schwefelsäure auf einen pH-Wert zwischen 1 und 3 gestellt und anschließend bei Temperaturen zwischen 50 und 100°C weiter umgesetzt.

Bei Verfahrensvariante b) erfolgt die Umsetzung in einem inerten organischen Lösungsmittel, beispielsweise einem aromatischen Lösungsmittel wie Toluol, Chlorbenzol, einem halogenierten Kohlenwasserstoff wie Chloroform oder in Dimethylformamid bei Temperaturen von 20 bis 120°C, vorzugsweise 60 bis 100°C.

Die Hydrolyse gemäß Verfahrensvariante c) erfolgt in Wasser, wäßriger Mineralsäure gegebenenfalls in Gegenwart eines inerten organischen Lösungsmittels bei Temperaturen zwischen 20 und 120°C, vorzugsweise 60 und 100°C. Als organische Lösungsmittel kommen beispielsweise mit Wasser nicht mischbare Lösungsmittel wie aromatische Lösungsmittel (z.B. Toluol, Chlorbenzol) oder halogenierte Kohlenwasserstoffe (z.B. Chloroform) in Betracht.

Die Verbindungen der Formel (II) sind bekannt oder lassen sich in Analogie zu bekannten Verfahren herstellen, s. Houben-Weyl, Methoden der organischen Chemie, Bd VIII, S. 120 (1952), Houben-Weyl Bd IX, S. 875, 869 (1955).

Amine der Formel III sind teilweise bekannt. Sie lassen sich für Q, M, T = $CR_2R_3$ und Q, T = $CR_2R_3$ und M = $NR_7$ durch einfache katalytische Hydrierung der entsprechenden Pyridin-oder Pyrazin-Derivate erhalten. Amine der For mel III können auch aus Aminen der Formel IV durch Umwandlung der Nitrilgruppe nach üblichen Methoden, s. z.B. Org. Snyth. Coll. Vol. I, S. 321 (1941) oder Houben-Weyl Bd VIII, S. 536,

hergestellt werden.

Amine der allgemeinen Formel IV sind teilweise bekannt gemäß JP-A 3073-569 oder lassen sich in analoger Weise nach dem dort beschrieben Verfahren herstellen.

Die erfindungsgemäßen Verbindungen der Formel I weisen eine ausgezeichnete herbizide Wirksamkeit gegen ein breites Spektrum wirtschaftlich wichtiger mono-und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf-oder Nachauflaufverfahren ausgebracht werden.

Beispielsweise können folgende Schadpflanzen bekämpft werden: Schadgräser wie Avena fatua, Alopecurus sp., Lolium sp., Setaria sp., Digitara sp., Sorghum halepense Echinochloa sp., Agropyron sp., Cynodon sp., Phalaris sp., dikotyle Pflanzen wie Lamium sp., Veronica sp. Galium sp., Stellaria sp., Matricaria sp., Papaver sp., Centauria sp., Amaranthus sp., Galinsoga sp., Mercurialis sp., Sida sp. Abutilon sp., Ambrosia sp., Xanthium sp., Cirsium sp., Artemisia sp., Rumex sp., Convolvulus sp., Ipomea sp., Sinapis sp..

Werden die erfindungsgemäßen Verbindungen vor dem Keimen auf die Erdoberfläche appliziert, so wird entweder das Auflaufen der Unkrautkeimlinge vollständig verhindert, oder dies Unkräuter wachsen bis zum Keimblattstadium heran, stellen jedoch dann ihr Wachstum ein und sterben schließlich nach Ablauf von drei bis fünf Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Verbindungen können, gegebenenfalls im Gemisch mit weiteren Wirkkomponenten, als Spritzpulver, emulgierbare Konzentrate, versprühbare Lösungen, Beizmittel, Stäubemittel, Dispersionen, Granulate oder Mikrogranulate in den üblichen Zugereitungen angewendet werden.

Spritzpulver sind in Wasser gleichmäßig dispergierbare Präparate, die neben dem (den) Wirkstoff(en) außer gegebenenfalls einem Verdünnungs-oder Inertstoff oder Netzmittel wie polyoxyethylierte Alkylphenole, polyoxethylierte Fettalkohole, Alkyl-oder Alkylphenylsulfonate und/oder Dispergierhilfsmittel wie ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen der Wirkstoffe in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Kondensationsprodukte, Alkylpolyglykolether, Sorbitanfettsäureester, Polyoxethylensorbitansäureester oder Polyoxethylensorbitester.

Stäubemittel kann man durch Vermahlen der Wirkstoffe mit feinverteilten, festen Stoffen, z.B. Talkum, natürlichen Tonen wie Kaolin, Bentonit, Pyrophillit oder Diatomeenerde erhalten.

Granulate können entweder durch Verdüsen der Wirkstoffe auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie

Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln -granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 0,05 bis 20 Gew.-% an Wirkstoff(en), versprühbare Lösungen etwa 2 bis 20 Gew.-%. Bei Granulaten hängt der Wirkstoffgehalt zum Teil davon-ab, ob die wirksame Verbindung flüssig oder fest vorliegt und welche Granulierhilfsmittel, Füllstoffe usw. verwendet werden.

Danebon enthalten die genannten Wirkstofformulierungen gegebenenfalls die jeweils üblichen Haft-, Netz-, Dispergier-, Emulgier-, Penetrations-, Lösemittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 2kg/ha.

Auch Mischungen der Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele erläutert.

## A. Formulierungsbeispiele

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether ( Triton X 207), 3 Gewichtsteilen Isotridecanolpolyglykolether (8 AeO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## Chemische Beispiele

### Beispiel 1

8-(4-Chlor-3-ethoxycarbonyl-phenyl)-7.9-dioxo-1.8-diazabicyclo[4.3.0]nonan-2-carbonsäure-(1-ethoxycarbonyl-ethylester)

37,3 g (0.10 mol) Piperidin-2.6-bis-carbonsäure-bis(1-ethoxycarbonyl-ethyl-ester) wurden in 200 ml Toluol gelöst. Dazu wurden bei 20 - 30°C 22,6 g (0,10 mol) 4-Chlor-3-ethoxycarbonyl-phenylisocyanat, gelöst in 50 ml Toluol, zugetropft.

Nach 1 h Rühren bei Raumtemperatur wurde noch 3 h bei 80 °C gerührt. Nach Abdestillieren des Lösemittels wurde der verbleibende Feststoff aus Methanol umkristallisiert. Man erhielt 39,5 g (82 % d. Th.) 8-(4-Chlor-3-ethoxycarbonyl-phenyl)-7.9-dioxo-1.8-diaza-bicyclo-[4.3.0] nonan-2-carbonsäure-(1-ethoxycarbonyl-ethylester) in Form farbloser Kristalle mit Schmelzpunkt: 121 - 128°C.

### Beispiel 2

8-(4-Chlor-2-fluor-5-methoxy-phenyl)-2-methyl-7.9-dioxo-1.8-diaza-bicyclo[4.3.0]nonan

Zu 17,1g(0,10 mol) 6-Methyl-piperidin-2-carbonsäureethylester in 100 ml Toluol wurden 20,2 g (0,10 mol) 4-Chlor-2-fluor-5-methoxy-phenylisocyanat, gelöst in 100 ml Toluol, zugetropft. Es wurde 3 h bei 100 °C gerührt. Das Lösemittel wurde unter vermindertem Druck abdestilliert und am Hochvakuum bis zur Gewichtskonstanz getrocknet.
Man erhielt 33.0 g (99 % d. Th.) 8-(4-Chlor-2-fluor-5-methoxy-phenyl)-2-methyl-7.9-di-oxo-1.8-diaza-bicyclo-[4.3.0]nonan in Form eines hellgelben Sirups.

Beispiel 3

8-(4-Chlor-2-fluor-5-methoxy-phenyl)-7.9-dioxo-1.8-diaza-bicyclo-[4.3.0]nonan-3-carbonsäureethylester

Zu 22,9 g (0,1 mol) Piperidin-2.5-dicarbonsäurediethylester in 150 ml Chlorbenzol wurden 20,2 g (0,1 mol) 4-Chlor-2-fluor-5-methoxy-phenylisocyanat, gelöst in 100 ml Chlorbenzol, zugetropft.
Es wurde 3 h bei 100 °C gerührt, das Lösungsmittel abdestilliert und am Hochvakuum bis zur Gewichtskonstanz getrocknet.
Man erhielt 37,8 g (98 % d. Th.) 8-(4-Chlor-2-fluor-5-methoxy-phenyl)-7.9-dioxo-1.8-diaza-bicyclo-[4.3.0]-nonan-3-carbonsäureethylester in Form eines honigfarbenen Sirups.

Beispiel 4

8-(4-Chlor-2-fluor-5-methoxy-phenyl)-7.9-dioxo-1.4.8-triazabicyclo[4.3.0]nonan-5-carbonsäureethylester

Zu 11,5 g (0,05 mol) Piperazin-2.3-dicarbonsäurediethylester in 100 ml Toluol wurden 10,1 g (0,05 mol) 4-Chlor-2-fluor-5-methoxyphenylisocyanat, gelöst in 70 ml Toluol, innerhalb von 1 h bei 20 °C zugetropft. Anschließend wurde 4 h bei 100 °C gerührt und das Lösungsmittel abdestilliert. Der verbleibende Rückstand wurde mit Hexan verrieben und abgesaugt. Man erhielt 14,8 g (77 % d. Th.) 8-(4-Chlor-2-fluor-5-methoxy-phenyl)-7.9-dioxo-1.4.8-triazabicyclo-[4.3.0]nonan-5-carbonsäureethylester in Form hellgelber Kristalle mit Schmelzpunkt: 55 - 58 °C.

Beispiel 5

8-(4-Chlorphenyl)-7-imino-9-oxo-1.8-diaza-bicyclo[4.3.0]nonan

Zu 11,0 g (0,1 mol) 2-Cyanopiperidin in 80 ml Toluol wurden 15.4 g (0,1 mol) 4-Chlorphenylisocyanat gelöst in 50 ml Toluol, getropft. Es wurde 4 h bei 100 °C gerührt und das Lösungsmittel abdestilliert.
Man erhielt 26,0 g (98 % d. Th.) 8-(4-Chlorphenyl)-7-imino-9-oxo-1.8-diaza-bicyclo[4.3.0]nonan in Form einer hellbraunen glasartigen Masse.

Beispiel 6

8-(4-Chlor-2-fluor-5-methoxy-phenyl)-3.5-dimethyl-7.9-dioxo-4-oxa-1.8-diaza-bicyclo[4.3.0]nonan

Zu 13.9 g (0,10 mol) 3-Cyano-2.7-dimethyl-morpholin in 100 ml Toluol wurden 20,2 g (0,10 mol) 4-Chlor-2-fluor-5-methoxyphenylisocyanat, gelöst in 100 ml Toluol, getropft. Es wurde 3 h bei 100 °C gerührt. Nach Zugabe von 50 ml 20 %iger wässriger Salzsäure wurde nochmals 2 h bei 100°C gerührt.
Die organische Phase wurde abgetrennt, 2 x mit 100 ml Wasser gewaschen und über Natriumsulfat getrocknet. Der nach dem Abziehen des Lösungsmittels verbleibende klebrige feste Rückstand wurde mit Di-isopropylether verrieben.
Man erhielt 26,4 g (77 % d. Th.) 8-(4-Chlor-2-fluor-5-methoxy-phenyl)-3.5-dimethyl-7.9-dioxo-4-oxa-1.8-diaza-bicyclo[4.3.0]nonan in Form farbloser Kristalle mit Schmelzpunkt: 126 - 131 °C.

Beispiel 7

8-(4-Chlor-2-fluor-5-methoxy-phenyl)-7.9-dioxo-3-thia-1.8-diaza-bicyclo[4.3.0]nonan

Zu 17,5 g (0,10 mol) 1.3-Thiazan-4-carbonsäureethylester in 150 ml Toluol wurden 20,2 (0,10 mol) 4-Chlor-2-fluor-5-methoxy-phenylisocyanat, gelöst in 50 ml Toluol, getropft. Es wurde 4 h bei 100 °C gerührt und das Lösungsmittel abdestilliert. Der zurückbleibende Feststoff wurde mit Di-isopropxylether verrieben und getrocknet.
Man erhielt 24,9 g (75 % d.Th.) 8-(4-Chlor-2-fluor-5-methoxy-phenyl)-7.9-dioxo-3-thia-1.8-diaza-bicyclo-[4.3.0]nonan in Form blaßgelber Kristalle mit Schmelzpunkt: 55 - 61 °C.

Beispiel 8

8-(4-Chlor-2-fluor-5-propargyloxy-phenyl)-2-(1.3-dioxolan-2-yl)-7.9-dioxo-1.8-diaza-bicyclo[4.3.0]nonan

Zu 11,5 g (0,05 mol) 6-(1.3-Dioxolan-2-yl)-piperidin-2-carbonsäureethylester in 100 ml Toluol wurden 11;3 g (0,05 mol) 4-Chlor-2-fluor-5-propargyloxy-phenylisocyanat, gelöst in 50 ml Toluol, zugetropft. Nach 3 h Rühren bei 100 °C wurde das Lösungsmittel abdestilliert und bis zur Gewichtskonstanz am Hochvakuum getrocknet.
Man erhielt 19,8 g (97 % d. Th.) 8-(4-Chlor-2-fluor-5-propargyloxy-phenyl)-2-(1.3-dioxolan-2-yl)-7.9-dioxo-1.8-diaza=bicyclo[4.3.0]nonan in Form eines farblosen Glases.

Beispiel 9

4-Benzyl-8-(4-Chlor-2-fluor-5-isopropoxy-phenyl)-7.9-dioxo-1.4.8-triaza-bicyclo[4.3.0]nonan

Zu 12,4 g (0,05 mol) 4-Benzyl-piperazin-2-carbonsäureethylester in 100 ml Toluol wurden 11,5 g (0,05 mol) 4-Chlor-2-fluor-5-isopropoxy-phenylisocyanat, gelöst in 50 ml Toluol, zugetropft.
Es wurde 3 h bei 100 °C gerührt und das Lösungsmittel abdestilliert.
Nach dem Trocknen am HV1) bis zur Gewichtskonstanz erhielt man 30,7 g (96 % d. Th.) 4-Benzyl-8-(4-Chlor-2-fluor-5-isopropoxy-phenyl)-7.9-dioxo-1.4.8-triaza-bicyclo[4.3.0]nonan in Form eines hellgelben Sirups.
HV = Hochvakuum

Beispiel 10

8-(4-Chlor-2-fluor-5-methoxy-phenyl)-7-oxo-9-thioxo-1.8-diaza-bicyclo[4.3.0]nonan-2-carbonsäure-(2.2.2-trifluorethylester)

Zu 16,9 g (0,05 mol) Piperidin-2.6-bis Carbonsäure (2.2.2-trifluorethyl)ester in 150 ml Toluol wurden 10,9g(0,05 mol) 4-Chlor-2-fluor-5-methoxy-phenylisothiocycanat, gelöst in 70 ml Toluol, zugetropft und 6 h bei 100 °C gerührt. Nach Abdestillieren des Lösungsmittels wurde der Rückstand mit n-Hexan verrieben und getrocknet.
Man erhielt 19,8 g (87 % d. Th.) 8-(4-Chlor-2-fluor-5-methoxy-phenyl)-7-oxo-9-thioxo-1.8-diaza-bicyclo-[4.3.0]nonan-2-carbonsäure-(2.2.2-tri-fluorethylester) in Form hellbeiger Kristalle mit Schmelzpunkt: 134 - 138 °C.

In analoger Weise lassen sich die Verbindungen (I) der folgenden Tabelle 1 herstellen.

## Tabelle 1

| Bsp. | Ar | X | Y | Q | M | T | $R_1$ | $R_2$ | $R_3$ | Fp($^oC$) |
|---|---|---|---|---|---|---|---|---|---|---|
| 11 | $4-Cl-C_6H_4$ | O | O | $-CH_2-$ | $-CH_2-$ | $-CH_2-$ | -H | 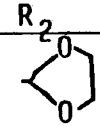 | -H | Glas |
| 12 | $4-Cl-2-F-5-OCH_3-C_6H_2$ | O | O | $-CH_2-$ | $-CH_2-$ | $-CH_2-$ | -H | $-CO_2C_2H_5$ | -H | Sirup |
| 13 | $4-Cl-2-F-S-OCH_3-C_6H_2$ | O | O | $-CH_2-$ | $-CH_2-$ | $-CH_2-$ | -H | $-CO_2CH_2CF_3$ | -H | 104-109 |
| 14 | $4-Cl-2-F-5-OCH_3-C_6H_2$ | O | O | $-CH_2-$ | $-CH_2-$ | $-CH_2-$ | -H | 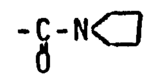 | -H | Glas |
| 15 | " | O | O | $-CH_2-$ | $-CH-CO_2C_2H_5$ | $-CH_2-$ | -H | -H | -H | Sirup |
| 16 | " | O | O | $=CH-CO_2C_2H_5$ | $-CH_2-$ | $-CH_2-$ | -H | -H | -H | Harz |
| 17 | " | O | O | $-CH_2-$ | $-CH_2-$ | $-CH_2-$ | -H | $-CO_2CH(CH_3)_2$ | -H | Glas |
| 18 | " | O | O | $-CH_2-$ | $-CH_2-$ | $-CH_2-$ | -H | $-CO_2CH_2CH_2-OCH_3$ | -H | Glas |
| 19 | " | O | O | $-CH_2-$ | $-CH_2-$ | $-CH_2-$ | -H | $-CO_2CH(CH_3)-CO_2C_2H_5$ | -H | Glas |
| 20 | " | O | O | $-CH_2-$ | $-CH_2-$ | $-CH_2-$ | -H | $-CH_2OH$ | -H | Harz |
| 21 | $4-Cl-2-F-5-OCH(CH_3)_2-C_6H_2$ | O | O | $-CH_2-$ | $-CH_2-$ | $=CH-C_2H_5$ | -H | -H | -H | Sirup |

...

| Bsp. | Ar | X | Y | Q | M | T | $R_1$ | $R_2$ | $R_3$ | Fp($^{o}$C) |
|---|---|---|---|---|---|---|---|---|---|---|
| 22 | $4-Cl-2F-5-OCH_3-C_6H_2$ | O | O | $-CH_2-$ | $-CH_2-$ | $-CH-CO_2C_2H_5$ | -H, | H | -H | Sirup |
| 23 | " | O | O | $-CH_2-$ | $-CH-CO_2C_2H_5$ | $-CH_2-$ | -H | $-CO_2C_2H_5$ | -H | Glas |
| 24 | $2,4-F_2-5-CO_2C_2H_5-C_6H_2-$ | O | O | $-CH_2-$ | $-CH_2-$ | $-CH_2-$ | -H | $-CH_3$ | -H | Sirup |
| 25 | $4-Br-3-CO_2-CH(CH_3)_2-C_6H_3$ | O | O | $-CH_2-$ | $-CH_2-$ | $-CH_2-$ | -H | $-CH_3$ | -H | Sirup |
| 26 | $4-Cl-3-CO_2CH(CH_3)CO_2C_2H_5-C_6H_2$ | O | O | $-CH_2$ | $-CH_2-$ | $-CH-CO_2CH_2$ $CH_3O$ | -H | -H | -H | Glas |
| 27 | $4-Cl-3-CO_2CH(CH_3)_2$ | O | O | $-CH-CO_2CH_3$ | $-CH_2-$ | $-CH_2-$ | -H | -H | -H | Glas |
| 28 | $4-Cl-2-F-5-CO_2C_2H_5-C_6H_2$ | O | O | $-CH_2-$ | $-CH_2-$ | $-CH_2-$ | -H | $-CO_2CH_3$ | -H | Sirup |
| 29 | $3-CO_2C_2H_5-4-OC_2H_5-C_6H_3$ | O | O | $-CH_2-$ | $-CH_2-$ | $-CH_2-$ | -H | $-CH_3$ | -H | Sirup |
| 30 | $3-CO_2C_2H_5-4-OCH_2CF_3-C_6H_3$ | O | O | $-CH_2-$ | $-CH_2-$ | $-CH_2-$ | -H | $-CO_2CH_2CF_3$ | -H | Glas |
| 31 | $2-F-4-OCF_3-5-OCH_3-C_6H_2$ | O | O | $-CH_2-$ | $-CH_2-$ | $-CH_2-$ | -H | $-CH_3$ | -H | Öl |
| 32 | $4-Cl-2-F-5-OCH_3-C_6H_2$ | S | O | $-CH_2-$ | $-CH_2-$ | $-CH_2-$ | -H | $-CH_3$ | -H | Harz |
| 33 | " | S | O | $-CH_2-$ | $-CH_2-$ | $-CH-C_2H_5$ | -H | -H | -H | Harz |
| 34 | " | S | O | $-CH-CO_2C_2H_5$ | $-CH_2$ | $-CH_2$ | -H | -H | -H | Harz |
| 35 | " | S | O | $-CH_2-$ | $-CH-CO_2CH_3$ | $-CH_2-$ | -H | -H | -H | Glas |

...

| Bsp. | Ar | X | Y | Q | M | T | $R_1$ | $R_2$ | $R_3$ | Fp(°C) |
|---|---|---|---|---|---|---|---|---|---|---|
| 36 | 4-Cl-2-F-5-$OCH_3$-$C_6H_2$ | S | O | $-CH_2-$ | $-CH_2-$ | $-CH_2-$ | -H | $-CO_2CH(CH_3)_2$ | -H | Glas |
| 37 | " | S | O | $-CH_2-$ | $-CH_2-$ | $-CH_2-$ | -H | $-CO_2CH_2CH_2OCH_3$ | -H | Sirup |
| 38 | 4-Br-3-$CO_2CH(CH_3)_2$-$C_6H_3$ | S | O | $-CH_2-$ | $-CH_2-$ | $-CH_2-$ | -H | $-CH_3$ | -H | Harz |
| 39 | 4-Cl-3-$CO_2CH(CH_3)CO_2C_2H_5$-$C_6H_3$ | S | O | $-CH_2-$ | $-CH_2-$ | $-CH-C_2H_5$ | -H | -H | -H | Sirup |
| 40 | 4-Cl-2-F-5-$OCH_2$-C≡CH-$C_6H_2$ | O | O | $-CH-CO_2C_2H_5$ | -NH- | $-CH_2-$ | -H | -H | -H | Harz |
| 41 | 4-Cl-2-F-5-$OCH(CH_3)_2$-$C_6H_2$ | O | O | $-CH_2-$ | $-N-CH_3$ | $-CH_2-$ | -H | -H | -H | Harz |
| 42 | 4-Cl-2-F-5-$OCH_3$-$C_6H_2$ | O | O | $-CH_2-$ | $-N-C(=O)-NHCH_3$ | $-CH_2-$ | -H | -H | -H | Glas |
| 43 | 4-Cl-2-F-5-$OCH_3$-$C_6H_2$ | O | O | $>CH-CO_2C_2H_5$ | 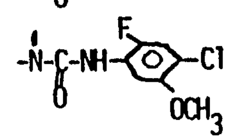 | $-CH_2-$ | -H | -H | -H | Glas |
| 44 | " | O | O | $-CH_2-$ | $-N-CO_2C_2H_5$ | $-CH_2-$ | -H | -H | -H | Sirup |
| 45 | " | O | O | $-CH_2-$ | $-N-C(=O)-CH_3$ | $-CH_2-$ | -H | -H | -H | Harz |
| 46 | " | O | O | $-CH-CO_2C_2H_5$ | $-N-CH_3$ | $-CH_2-$ | -H | -H | -H | Harz |
| 47 | " | O | O | $-CH-CO_2C_2H_5$ | $-N-CO_2CH_3$ | $-CH_2-$ | -H | -H | -H | Harz |

...

| Bsp. | Ar | X | Y | Q | M | T | $R_1$ | $R_2$ | $R_3$ | Fp($^{o}C$) |
|---|---|---|---|---|---|---|---|---|---|---|
| 48 | $4\text{-}Br\text{-}5\text{-}CO_2C_2H_5\text{-}C_6H_3$ | 0 | 0 | $-CH-CO_2C_2H_5$ | -NH | $-CH_2-$ | -H | -H | -H | Harz |
| 49 | $4\text{-}Cl\text{-}5\text{-}CO_2CH(CH_3)CO_2C_2H_5\text{-}C_6H_3$ | 0 | 0 | $-CH-CO_2C_2H_5$ | $-N-CH_3$ | $-CH_2-$ | -H | -H | -H | Harz |
| 50 | $4\text{-}Cl\text{-}2\text{-}F\text{-}5\text{-}OCH_3\text{-}C_6H_2$ | S | 0 | $-CH-CO_2C_2H_5$ | -NH | $-CH_2-$ | -H | -H | -H | Glas |
| 51 | " | S | 0 | $-CH_2-$ | $-N-CH_3$ | $-CH_2-$ | -H | -H | -H | Sirup |
| 52 | " | S | 0 | $-CH-CO_2C_2H_5$ | $-N-CO_2CH_3$ | $-CH_2-$ | -H | -H | -H | Harz |
| 53 | " | S | 0 | $-CH_2-$ | $-N-C(=O)-CH_3$ | $-CH_2-$ | -H | -H | -H | Harz |
| 54 | " | 0 | 0 | $-CH_2-$ | -0- | $-CH_2-$ | -H | -H | -H | Sirup |
| 55 | $4\text{-}Cl\text{-}C_6H_4$ | 0 | 0 | $-CH_2-$ | -0- | $-CH_2-$ | -H | -H | -H | 121-125 |
| 56 | $4\text{-}Br\text{-}3\text{-}CO_2C_2H_5\text{-}C_6H_3$ | 0 | 0 | $-CH_2-$ | -0- | $-CH_2-$ | -H | -H | -H | Sirup |
| 57 | $4\text{-}Cl\text{-}3\text{-}CO_2CH(CH_3)CO_2C_2H_5\text{-}C_6H_3$ | 0 | 0 | $-CH_2-$ | -0- | $-CH_2-$ | -H | -H | -H | Harz |
| 58 | $4\text{-}Cl\text{-}2\text{-}F\text{-}5\text{-}OCH_3\text{-}C_6H_2$ | S | 0 | $-CH_2$ | -0- | $-CH_2$ | -H | -H | -H | 97-102 |
| 59 | " | S | 0 | $-CH-CH_3$ | -0- | $-CH-CH_3$ | -H | -H | -H | Glas |
| 60 | $4\text{-}Cl\text{-}C_6H_4$ | 0 | 0 | $-CH_2-$ | -S- | $-CH_2-$ | -H | $-CH_3$ | -H | Sirup |

...

| Bsp. | Ar | X | Y | Q | M | T | $R_1$ | $R_2$ | $R_3$ | Fp($^oC$) |
|---|---|---|---|---|---|---|---|---|---|---|
| 61 | 4-Cl-2-F-5-$OCH_3$-$C_6H_2$ | O | O | -CH-$CH_3$ | -S- | -CH-$CH_3$ | -H | -H | -H | Harz |
| 62 | " | S | O | -CH-$CH_3$ | -S- | -CH-$CH_3$ | -H | -H | -H | Harz |
| 63 | " | O | NH | -$CH_2$- | -$CH_2$- | -$CH_2$- | -H | -H | -H | 48-53 |
| 64 | 4-Br-2F-5-$OCH_3$-$C_6H_2$ | O | NH | -$CH_2$- | -$CH_2$- | -$CH_2$- | -H | -H | -H | 75-80 |
| 65 | 4-Cl-3-$CO_2CH(CH_3)CO_2C_2H_5$-$C_6H_3$ | O | NH | -$CH_2$- | -$CH_2$- | -$CH_2$- | -H | -H | -H | Sirup |
| 66 | 3-5-$Cl_2$-4-$CF_3CHFCF_2O$-$C_6H_2$ | O | NH | -$CH_2$- | -$CH_2$- | -$CH_2$- | -H | -H | -H | 102-106 |
| 67 | 4-Cl-2-F-5-$OCH_3$-$C_6H_2$ | O | NH | -$CH_2$- | -CH-$CH_3$ | -$CH_2$ | -H | -H | -H | Glas |
| 68 | " | O | NH | -$CH_2$- | -O- | -$CH_2$- | -H | -H | -H | 142-147 |
| 69 | 4-Cl-2F-5-$OCH_2C{\equiv}CH$-$C_6H_2$ | O | NH | -$CH_2$- | -$CH_2$- | -$CH_2$- | -H | -H | -H | 112-116 |
| 70 | 4-Cl-2F-5-$OCH(CH_3)_2$-$C_6H_2$ | O | NH | -$CH_2$- | -O- | -$CH_2$- | -H | -H | -H | Glas |
| 71 | 4-Br-3-$CO_2CH(CH_3)_2$-$C_6H_3$ | O | NH | -$CH_2$- | -$CH_2$- | -$CH_2$- | -H | -H | -H | 86-90 |
| 72 | 4-Cl-2-F-5-$CO_2C_2H_5$-$C_6H_2$ | O | NH | -$CH_2$- | -O- | -$CH_2$- | -H | -H | -H | Glas |
| 73 | 4-$OCH_3$-3-$CO_2C_2H_5$-$C_6H_3$ | O | NH | -$CH_2$- | -$CH_2$- | -$CH_2$- | -H | -H | -H | Harz |
| 74 | 4-S-$C_2H_5$-3-$CO_2CH(CH_3)_2$-$C_6H_3$ | O | NH | -$CH_2$- | -$CH_2$- | -$CH_2$- | -H | -H | -H | Harz |

...

| Bsp. | Ar | X | Y | Q | M | T | $R_1$ | $R_2$ | $R_3$ | Fp($^oC$) |
|---|---|---|---|---|---|---|---|---|---|---|
| 75 | $4-OCH_2CF_3-3-CO_2C_2H_5-C_6H_3$ | O | NH | $-CH_2-$ | -O- | $-CH_2-$ | -H | -H | -H | Harz |
| 76 | $4-Cl-2-F-5-OCH_3-C_6H_2$ | O | NH | $-CH_2-$ | $-CH-CH_3$ | $-CH_2-$ | -H | -H | -H | Glas |
| 77 | " | O | NH | $-CH-CH_3$ | $-CH_2-$ | $-CH-CH_3$ | -H | -H | -H | Glas |
| 78 | " | O | NH | $-CH_2-$ | $-CH_2-$ | $-CH_2-$ | $CH_3$ | $-CH_3$ | -H | Sirup |
| 79 | " | O | NH | $-CH_2-$ | $-CH-CH_3$ | $-CH_2$ | $-CH_3$ | $-CH_3$ | -H | Sirup |
| 80 | " | O | NH | $-CH_2-$ | -O- | $-CH_2-$ | $-CH_3$ | $-CH_3$ | -H | Harz |
| 81 | " | O | NH | $-CH_2-$ | -NH- | $-CH_2-$ | -H | -H | -H | Harz |
| 82 | " | O | NH | $-CH_2-$ | $-N-CH_3$ | $-CH_2-$ | -H | -H | -H | Sirup |
| 83 | " | O | NH | $-CH_2-$ | $-N-C_6H_5$ | $-CH_2-$ | -H | -H | -H | Harz |
| 84 | " | O | NH | $-CH_2-$ | $-N-C(=O)-NHCH_3$ | $-CH_2-$ | -H | -H | -H | Harz |
| 85 | " | O | NH | $-CH_2-$ | $-N-P(=O)(OC_2H_5)_2$ | $-CH_2-$ | -H | -H | -H | Glas |
| 86 | " | S | NH | $-CH_2-$ | $-CH-CH_3$ | $-CH_2-$ | -H | -H | -H | Gals |
| 87 | $4-Cl-2-F-5-OCH_2C\equiv CH-C_6H_2$ | S | NH | $-CH_2-$ | $-CH_2-$ | $-CH_2-$ | -H | -H | -H | Glas |
| 88 | $4-Cl-2-F-5-OCH(CH_3)_2-C_6H_2$ | S | NH | $-CH-CH_3$ | $-CH_2-$ | $-CH-CH_3$ | -H | -H | -H | Harz |

...

| Bsp. | Ar | X | Y | Q | M | T | $R_1$ | $R_2$ | $R_3$ | Fp($^oC$) |
|---|---|---|---|---|---|---|---|---|---|---|
| 89 | 4-Cl-2-F-5-$OCH_3$-$C_6H_5$ | S | NH | -$CH_2$- | -O- | -$CH_2$- | -H | -H | -H | Glas |
| 90 | " | S | NH | -CH-$CH_3$ | -O- | -CH-$CH_3$ | -H | -H | -H | Glas |
| 91 | " | S | NH | -$CH_2$- | -S- | -$CH_2$- | -H | -H | -H | Sirup |
| 92 | " | S | NH | -$CH_2$- | -CH-$CO_2C_2H_5$ | -$CH_2$ | -H | -H | -H | Sirup |
| 93 | " | S | NH | -$CH_2$- | -NH- | -$CH_2$- | -H | H | -H | Harz |
| 94 | " | S | NH | -$CH_2$- | -N-$CH_3$ | -$CH_2$- | -H | -H | -H | Harz |
| 95 | " | S | NH | -$CH_2$- | -$CH_2$- | -S- | -H | -H | -H | Harz |
| 96 | " | S | O | -$CH_2$- | -$CH_2$- | -S- | -H | -H | -H | Harz |
| 97 | 4-Br-2-F-5-$OCH_3$-$C_6H_2$ | S | O | -$CH_2$- | -$CH_2$- | -S- | -H | -H | -H | Harz |
| 98 | 4-Br-2F-5-$OCH_2C{\equiv}CH$-$C_6H_2$ | O | O | -$CH_2$- | -$CH_2$- | -S- | -H | -H | -H | Harz |
| 99 | 4-Cl-2F-5$OCH(CH_3)_2$-$C_6H_2$ | O | O | -$CH_2$- | -$CH_2$- | -S- | -H | -H | -H | Harz |
| 100 | 4-Cl-2F-5-$OCH_2C{\equiv}CH$-$C_6H_2$ | S | O | -$CH_2$- | -$CH_2$- | -S- | -H | -H | -H | Sirup |
| 101 | 4-Br-5-$CO_2CH(CH_3)_2$-$C_6H_3$ | S | O | -$CH_2$- | -$CH_2$- | -S- | -H | -H | -H | Harz |
| 102 | 4-Cl-5-$CO_2CH(CH_3)CO_2C_2H_5$-$C_6H_3$ | O | O | -$CH_2$- | -$CH_2$- | -S- | -H | -H | -H | Harz |
| 103 | 4-$OCH_2CF_3$-3-$CO_2C_2H_5$-$C_6H_3$ | O | O | -$CH_2$- | -$CH_2$- | -S- | -H | -H | -H | Harz |
| 104 | 2,4-$F_2$-5-$CO_2CH(CH_3)_2$-$C_6H_2$ | O | O | -$CH_2$- | -$CH_2$- | -S- | -H | -H | -H | Sirup |
| 105 | 4-$OC_2H_5$-3-$CO_2C_2H_5$-$C_6H_3$ | S | O | -$CH_2$- | -$CH_2$- | -S- | -H | -H | -H | Sirup |
| 106 | 4-$SC_2H_5$-3-$CO_2CH_2CF_3$-$C_6H_3$ | O | O | -$CH_2$- | -$CH_2$- | -S- | -H | -H | -H | Harz |

...

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:
0 = ohne Wirkung
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

**1. Unkrautwirkung im Vorauflauf**

Samen bzw. Rhizomstücke von mono-und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsiosnen mit einer Wasseraufwandmenge von umgerechnet 600 - 800 l/ha in unterschiedlichen dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen-bzw. der Auflaufschäden erfolgte nach dem Auflaufen der Versuchspflanzen nach einer Versuchszeit von 3 - 4 Wochen im Vergleich zu unbehandelten Kontrollen. Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono-und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von ungerechnet 600 - 800 l/ha auf die grünen Pflanzenteile gesprüht und nach ca. 3 - 4 Wochen Standzeit der Versuchspflanzen im Gewächshaus unter optimale Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungrässer und Unkräuter auf (Tabelle 3).

Tabelle 2

Vorauflaufwirkung

| Beispiel-Nr. | Dosis kg a.i./ha | herbizide Wirkung STM | CRS | SIA | LOM | ECG |
|---|---|---|---|---|---|---|
| 2 | 2,5 | 5 | 5 | 5 | 5 | 5 |
| 5 | 2,5 | 3 | 4 | 2 | 2 | 4 |
| 6 | 2,5 | 5 | 5 | 5 | 5 | 5 |
| 7 | 2,5 | 5 | 5 | 5 | 5 | 5 |
| 54 | 2,5 | 5 | 5 | 5 | 5 | 5 |
| 55 | 2,5 | 5 | 5 | 5 | 5 | 5 |
| 63 | 2,5 | 5 | 5 | 5 | 5 | 5 |
| 64 | 2,5 | 5 | 5 | 5 | 5 | 5 |
| 67 | 2,5 | 5 | 5 | 5 | 5 | 5 |
| 68 | 2,5 | 5 | 5 | 5 | 5 | 5 |

Tabelle 3

Nachauflaufwirkung

| Beispiel-Nr. | Dosis kg a.i./ha | herbizide Wirkung STM | CRS | SIA | LOM | ECG |
|---|---|---|---|---|---|---|
| 2 | 2,5 | 5 | 5 | 5 | 5 | 5 |
| 6 | 2,5 | 5 | 4 | 5 | 5 | 5 |
| 7 | 2,5 | 5 | 4 | 5 | 5 | 4 |
| 54 | 2,5 | 5 | 5 | 5 | 5 | 5 |
| 55 | 2,5 | 4 | 3 | 3 | 3 | 4 |
| 63 | 2,5 | 5 | 4 | 5 | 5 | 3 |
| 64 | 2,5 | 4 | 3 | 5 | 4 | 4 |
| 67 | 2,5 | 4 | 5 | 5 | 3 | 3 |
| 68 | 2,5 | 5 | 5 | 5 | 5 | 5 |

Abkürzungen:

STM = Stellaria media
CRS = Chrysanthemum segetum
SIA = Sinapis alba
LOM = Lolium multiflorum
ECG = Echinochloa crus-galli
a.i. = Aktivsubstanz

**Ansprüche**

1. Verbindungen der Formel I oder deren Salze,

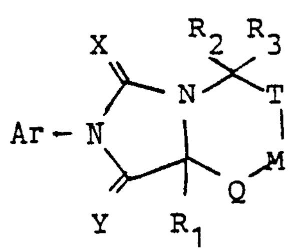

(I),

worin

Ar = Phenyl, Naphthyl, Pyridyl, Chinolinyl oder Isochinolinyl bedeuten, wobei diese Reste ein-bis vierfach, vorzugsweise ein-bis dreifach, durch gleiche oder verschiedene Reste der Gruppe Halogen, Hydroxy, $(C_1-C_4)$Alkyl, Halo$(C_1-C_4)$alkyl, $(C_1-C_4)$Alkoxy, $(C_3-C_6)$-Cycloalkoxy, $(C_3-C_6)$Alkenyloxy, $(C_3-C_6)$Alkinyloxy, $(C_1-C_4)$-Alkoxy-$(C_1-C_4)$alkoxy, Halo$(C_1-C_4)$alkoxy, Halo$(C_3-C_6)$alkenyloxy, Halo$(C_3-C_6)$alkinyloxy, $(C_1-C_4)$Alkylthio, $(C_1-$

$C_4$)Alkylsulfinyl, ($C_1$-$C_4$)Alkylsulfonyl, $NO_2$, -CN, -$NHR_1$, Cyano($C_1$-$C_4$)alkyl, Phenoxy, Phenoxy($C_1$-$C_4$)alkyl, Phenyl($C_1$-$C_4$)alkoxy, Phenylthio, Phenylsulfinyl, Phenylsulfonyl, wobei die sechs letztgenannten Reste im Phenylring bis zu dreifach durch Halogen, ($C_1$-$C_4$)Alkyl, ($C_1$-$C_4$)Alkoxy, ($C_1$-$C_4$)Alkoxycarbonyl, -CN oder $NO_2$ substituiert sein können, oder ferner durch einen Rest der Formeln

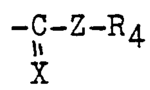 , 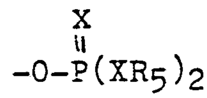 oder 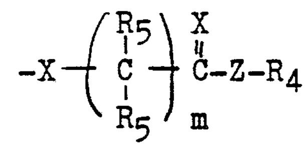

substituiert sein können;

$R_1$ Wasserstoff, ($C_1$-$C_4$)Alkyl oder Phenyl, das bis zu zweifach durch Halogen, ($C_1$-$C_4$)Alkyl, ($C_1$-$C_4$)Alkoxy, $NO_2$, CN oder ($C_1$-$C_4$)Alkoxycarbonyl substituiert sein kann,

$R_2$,$R_3$ unabhängig voneinander Wasserstoff, ($C_1$-$C_4$)Alkyl, das durch Cyano, Hydroxy oder ($C_1$-$C_4$)Alkoxy substituiert sein kann, Phenyl oder Benzyl, die beide im Phenylring jeweils vorzugsweise bis zu 2-fach durch Halogen, ($C_1$-$C_4$)Alkyl, ($C_1$-$C_4$)Alkoxy, $NO_2$ oder -CN substituiert sein können;

($C_1$-$C_4$)Alkoxy, ($C_1$-$C_4$)Alkoxycarbonyl, Halogen($C_1$-$C_4$)alkoxycarbonyl, ($C_1$-$C_4$)-Alkoxy($C_1$-$C_4$)alkoxycarbonyl, ( $C_1$-$C_4$)Alkoxycarbonyl($C_1$-$C_4$)alkoxycarbonyl, Carboxy, oder einen Rest der Formeln

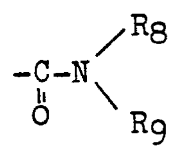 , 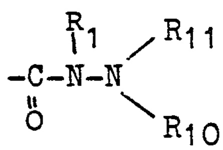 , 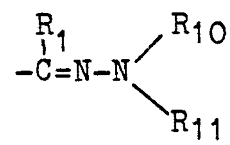 oder

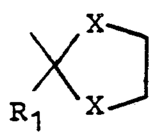

$R_4$ Wasserstoff, ($C_1$-$C_4$)Alkyl, das bis zu sechsfach durch Halogen und/oder bis zu zweifach durch ($C_1$-$C_4$)-Alkoxy, ($C_1$-$C_4$)Alkoxy($C_1$-$C_4$)alkoxy, ($C_1$-$C_4$)Alkoxycarbonyl, ($C_1$-$C_4$)Alkylthio, ($C_1$-$C_4$)Alkylsulfinyl, ($C_1$-$C_4$)-Alkylsulfonyl, ($C_1$-$C_4$)Alkylamino, -CN, Furyl, Tetrahydrofuryl, Benzofuryl, Phenyl, Phenoxy, Benzyloxy, wobei bei den sechs letztgenannten Resten jeweils der Phenylring oder der Heteroaromat bis zu dreifach durch Halogen, ($C_1$-$C_4$)Alkyl oder ($C_1$-$C_4$)Alkoxy substituiert sein kann, ($C_3$-$C_6$)Cycloalkyl, ($C_3$-$C_6$)Alkenyl, Cyclohexenyl, ($C_3$-$C_6$)Alkinyl, Phenyl, das bis zu dreifach substituiert sein kann durch Halogen, ($C_1$-$C_4$)Alkyl, ($C_1$-$C_4$)-Alkoxy oder ($C_1$-$C_4$)Alkoxycarbonyl; oder einen Rest der Formel

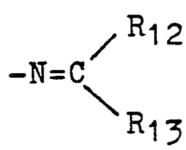

wobei der letztgenannte Rest für Z = S ausgenommen ist,

$R_5$ Wasserstoff oder ($C_1$-$C_4$)Alkyl,

$R_6$ Wasserstoff, ($C_1$-$C_4$)Alkyl oder zusammen mit $R_4$ und dem diese Reste verbindenden Stickstoffatom einen 5-bis 7-gliedrigen Heterocyclus, der als Ringglieder ein oder zwei Reste der Gruppe -O-, -S-, -$NR_5$- enthalten kann und bis zu 3-fach durch ($C_1$-$C_4$)Alkyl substituiert sein kann;

$R_7$ Wasserstoff, ($C_1$-$C_4$)Alkyl, Phenyl oder Benzyl, wobei der Phenylring jeweils bis zu dreifach substituiert sein kann durch Halogen, ($C_1$-$C_4$)Alkyl, ($C_1$-$C_4$)Alkoxy, ($C_1$-$C_4$)Alkoxycarbonyl, -$NO_2$, $CF_3$, -CN oder einen Rest der Formeln

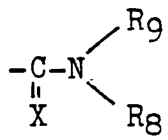 , 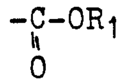 , 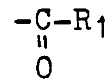 , 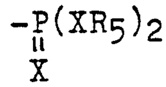 oder

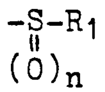

$R_8$,$R_9$ unabhängig voneinander Wasserstoff oder ($C_1$-$C_4$)Alkyl oder beide Reste $R_8$, $R_9$ zusammen mit den sie verbindenden Stickstoffatom einen 5-bis 7-gliedrigen Heterocyclus, der als Ringglieder ein oder zwei Reste der Gruppe -O-, -S0, -$NR_6$-enthalten kann und der bis zu dreifach durch ($C_1$-$C_4$)Alkyl, Hydroxy, ($C_1$-$C_4$)-Alkoxy, Phenyl oder Benzyl, die beide im Phenylring, vorzugsweise bis zu dreifach, durch ($C_1$-$C_4$)Alkyl, ($C_1$-$C_4$)Alkoxy, Halogen oder ($C_1$-$C_4$)Alkoxycarbonyl substituiert sein können, substituiert sein kann,
$R_{10}$,$R_{11}$ unabhängig voneinander Wasserstoff, ($C_1$-$C_4$)Alkyl, Phenyl oder Benzyl, die beide jeweils im Phenylring, vorzugsweise bis zu dreifach, substituiert sein können durch Halogen, ($C_1$-$C_4$)Alkyl, ($C_1$-$C_4$)-Alkoxy, $NO_2$, -CN, $CF_3$ oder ($C_1$-$C_4$)Alkoxycarbonyl, oder $R_{10}$ und $R_{11}$ zusammen den Rest

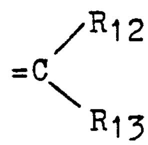 ,

$R_{12}$,$R_{13}$ unabhängig voneinander, ($C_1$-$C_4$)Alkyl, ($C_1$-$C_4$)Alkoxycarbonyl, Phenyl oder Benzyl, die beide im Phenylring, vorzugsweise bis zu dreifach, substituiert sein können durch Halogen, ($C_1$-$C_4$)Alkyl, ($C_1$-$C_4$)-Alkoxy, ($C_1$-$C_4$)Alkoxycarbonyl, $CF_3$, -CN oder $NO_2$,

M 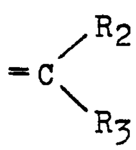 , -S-, 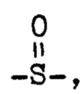, 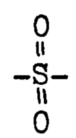 , -O- oder $NR_7$ ;

Q 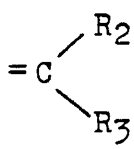 , -S- oder -O-

T 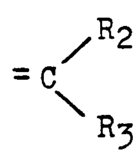 , -S-, 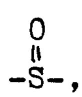, 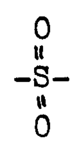 oder -O- ,

X = O oder S
Y = O, S oder NH,
Z = O, S oder $NR_6$,
m = 1, 2, 3 und
n = 1 oder 2 bedeuten,
mit der Maßgabe, daß, wenn Y = O, $R_1$, $R_2$ , $R_3$ = H und Q, T = $CH_2$ bedeuten, M nicht $CH_2$, S, SO oder $SO_2$ sein darf.

2. Verbindungen der Formel I von Anspruch 1, worin
Ar = Phenyl, das bis zu dreifach substituiert sein kann durch Fluor, Chlor oder Brom, ($C_1$-$C_4$)Alkyl, ($C_1$-$C_4$)-Alkoxy, ($C_3$-$C_4$)Alkenyloxy, ($C_3$-$C_4$)Alkinyloxy, Halo($C_1$-$C_4$)alkyl, Halo($C_1$-$C_4$)alkoxy, -$NHR_1$, ($C_1$-$C_4$)Alkylthio, ($C_1$-$C_4$)Alkylsulfonyl, $NO_2$ oder einen Rest der Formeln

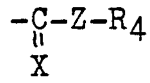 oder 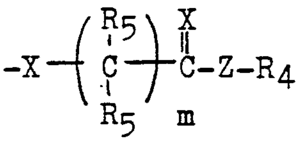

$R_1$ = Wasserstoff, $(C_1\text{-}C_4)$Alkyl, $(C_1\text{-}C_4)$Alkoxycarbonyl

$R_2$,$R_3$ = unabhängig voneinander Wasserstoff, $(C_1\text{-}C_4)$Alkyl, $(C_1\text{-}C_4)$Alkoxycarbonyl, $(C_1\text{-}C_4)$Alkoxy$(C_1\text{-}C_4)$-alkoxycarbonyl, Halogen$(C_1\text{-}C_4)$alkoxycarbonyl, $(C_1\text{-}C_4)$Alkoxycarbonyl$(C_1\text{-}C_4)$alkoxycarbonyl, -CN,Carboxy,

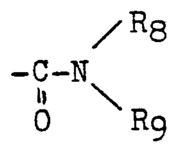

$R_4$ = $(C_1\text{-}C_4)$Alkyl, Halogen$(C_1\text{-}C_4)$alkyl, $(C_1\text{-}C_4)$Alkoxyalkyl, $(C_1\text{-}C_4)$Alkoxycarbonyl$(C_1\text{-}C_4)$alkyl;

$R_5$ = H oder $(C_1\text{-}C_4)$Alkyl,

$R_7$ = Wasserstoff, $(C_1\text{-}C_4)$Alkyl,

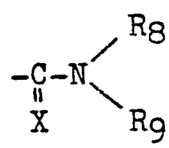 ;

Phenyl oder Benzyl, die beide bis zu zweifach durch $(C_1\text{-}C_4)$Alkyl, $(C_1\text{-}C_4)$Alkoxy, Halogen, $NO_2$, oder $-CF_3$ substituiert sein können,

$R_8$,$R_9$ = Wasserstoff, $(C_1\text{-}C_4)$Alkyl, zusammen mit den sie verbindenden N-Atom einem 6-gliedrigen gesättigten Heterocyclus

M = 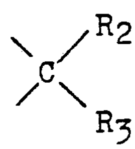 , -O- oder $-NR_7-$

Q = 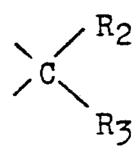

T = 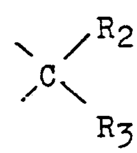 oder -S-

X = O oder S,

Y = O oder NH,

Z = O oder S und

m = 1 bedeuten.

3. Verfahren zur Herstellung der Verbindungen der Formel I von Anspruch 1 oder 2 oder deren Salze, dadurch gekennzeichnet, daß man

a) für Y = O, S eine Verbindung der Formel II mit einer Verbindung der Formel III,

$Ar-N=C=X$

II

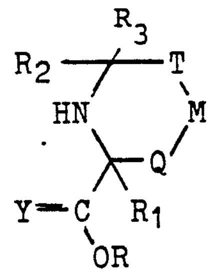

III

worin R = H oder ($C_1$-$C_4$)Alkyl bedeutet, oder

b) eine Verbindung der Formel II mit einem Amin der Formel IV umsetzt

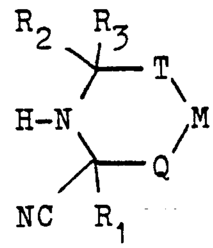

(IV)

oder

c) für Y = O eine unter b) erhaltene Verbindung der Formel I hydrolysiert und die erhaltenen Verbindungen gegebenenfalls in ihre Salze überführt.

4. Herbizide Mittel, gekennzeichnet durch einen Gehalt an einer Verbindung der Formel I von Ansprüchen 1 oder 2 oder deren Salz.

5. Verwendung der Verbindungen der Formel I von Ansprüchen 1 oder 2 oder deren Salz als Herbizide.

6. Verfahren zur Bekämpfung von unerwünschten Pflanzen, dadurch gekennzeichnet, daß man eine wirksame Menge einer Verbindung der Formel I oder deren Salz von Ansprüchen 1 oder 2 auf diese oder die landwirtschaftlich genutzten Böden appliziert.

Patentansprüche für den folgenden Vertragsstaat: ES

1. Verfahren zur Herstellung der Verbindungen der Formel I oder deren Salze,

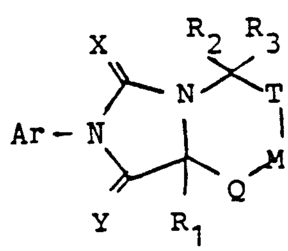

(I),

worin

Ar = Phenyl, Naphthyl, Pyridyl, Chinolinyl oder Isochinolinyl bedeuten, wobei diese Reste ein-bis vierfach, vorzugsweise ein-bis dreifach, durch gleiche oder verschiedene Reste der Gruppe Halogen, Hydroxy, ($C_1$-$C_4$)Alkyl, Halo($C_1$-$C_4$)alkyl, ($C_1$-$C_4$)Alkoxy, ($C_3$-$C_6$)-Cycloalkoxy, ($C_3$-$C_6$)Alkenyloxy, ($C_3$-$C_6$)Alkinyloxy, ($C_1$-$C_4$)-Alkoxy-($C_1$-$C_4$)alkoxy, Halo($C_1$-$C_4$)alkoxy, Halo($C_3$-$C_6$)alkenyloxy, Halo($C_3$-$C_6$)alkinyloxy, ($C_1$-$C_4$)Alkylthio, ($C_1$-$C_4$)Alkylsulfinyl, ($C_1$-$C_4$)Alkylsulfonyl, $NO_2$, -CN, -$NHR_1$, Cyano($C_1$-$C_4$)alkyl, Phenoxy, Phenoxy($C_1$-$C_4$)alkyl, Phenyl($C_1$-$C_4$)alkoxy, Phenylthio, Phenylsulfinyl, Phenylsulfonyl, wobei die sechs letztgenannten Reste im Phenylring bis zu dreifach durch Halogen, ($C_1$-$C_4$)Alkyl, ($C_1$-$C_4$)Alkoxy, ($C_1$-$C_4$)Alkoxycarbonyl, -CN oder $NO_2$ substituiert sein können, oder ferner durch einen Rest der Formeln

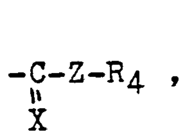 , 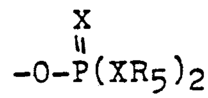 oder 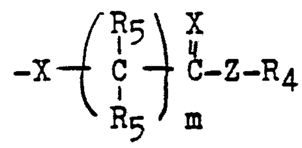

substituiert sein können;

$R_1$ Wasserstoff, $(C_1-C_4)$Alkyl oder Phenyl, das bis zu zweifach durch Halogen, $(C_1-C_4)$Alkyl, $(C_1-C_4)$Alkoxy, $NO_2$, CN oder $(C_1-C_4)$Alkoxycarbonyl substituiert sein kann,

$R_2$,$R_3$ unabhängig voneinander Wasserstoff, $(C_1-C_4)$Alkyl, das durch Cyano, Hydroxy oder $(C_1-C_4)$Alkoxy substituiert sein kann, Phenyl oder Benzyl, die beide im Phenylring jeweils vorzugsweise bis zu 2-fach durch Halogen, $(C_1-C_4)$Alkyl, $(C_1-C_4)$Alkoxy, $NO_2$ oder -CN substituiert sein können;

$(C_1-C_4)$Alkoxy, $(C_1-C_4)$Alkoxycarbonyl, Halogen$(C_1-C_4)$alkoxycarbonyl, $(C_1-C_4)$-Alkoxy$(C_1-C_4)$alkoxycarbonyl, ( $C_1-C_4)$Alkoxycarbonyl$(C_1-C_4)$alkoxycarbonyl, Carboxy, oder einen Rest der Formeln

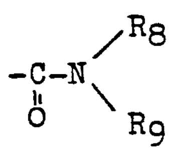 , 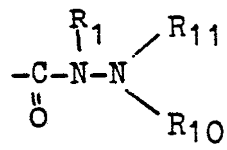 , 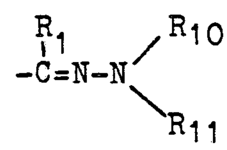 oder

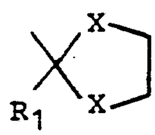

$R_4$ Wasserstoff, $(C_1-C_4)$Alkyl, das bis zu sechsfach durch Halogen und/oder bis zu zweifach durch $(C_1-C_4)$-Alkoxy, $(C_1-C_4)$Alkoxy$(C_1-C_4)$alkoxy, $(C_1-C_4)$Alkoxycarbonyl, $(C_1-C_4)$Alkylthio, $(C_1-C_4)$Alkylsulfinyl, $(C_1-C_4)$-Alkylsulfonyl, $(C_1-C_4)$Alkylamino, -CN, Furyl, Tetrahydrofuryl, Benzofuryl, Phenyl, Phenoxy, Benzyloxy, wobei bei den sechs letztgenannten Resten jeweils der Phenylring oder der Heteroaromat bis zu dreifach durch Halogen, $(C_1-C_4)$Alkyl oder $(C_1-C_4)$Alkoxy substituiert sein kann, $(C_3-C_6)$Cycloalkyl, $(C_3-C_6)$Alkenyl, Cyclohexenyl, $(C_3-C_6)$Alkinyl, Phenyl, das bis zu dreifach substituiert sein kann durch Halogen, $(C_1-C_4)$Alkyl, $(C_1-C_4)$-Alkoxy oder $(C_1-C_4)$Alkoxycarbonyl; oder einen Rest der Formel

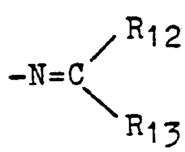

wobei der letztgenannte Rest für Z = S ausgenommen ist,

$R_5$ Wasserstoff oder $(C_1-C_4)$Alkyl,

$R_6$ Wasserstoff, $(C_1-C_4)$Alkyl oder zusammen mit $R_4$ und dem diese Reste verbindenden Stickstoffatom einen 5-bis 7-gliedrigen Heterocyclus, der als Ringglieder ein oder zwei Reste der Gruppe -O-, -S-, $-NR_5-$ enthalten kann und bis zu 3-fach durch $(C_1-C_4)$Alkyl substituiert sein kann;

$R_7$ Wasserstoff, $(C_1-C_4)$Alkyl, Phenyl oder Benzyl, wobei der Phenylring jeweils bis zu dreifach substituiert sein kann durch Halogen, $(C_1-C_4)$Alkyl, $(C_1-C_4)$Alkoxy, $(C_1-C_4)$Alkoxycarbonyl, $-NO_2$, $CF_3$, -CN oder einen Rest der Formeln

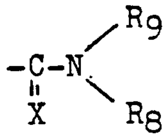 , 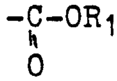 , 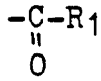 , 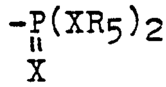 oder

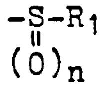

$R_8$,$R_9$ unabhängig voneinander Wasserstoff oder ($C_1$-$C_4$)Alkyl oder beide Reste $R_8$, $R_9$ zusammen mit den sie verbindenden Stickstoffatom einen 5-bis 7-gliedrigen Heterocyclus, der als Ringglieder ein oder zwei Reste der Gruppe -O-, -S-, -$NR_5$-enthalten kann und der bis zu dreifach durch ($C_1$-$C_4$)Alkyl, Hydroxy, ($C_1$-$C_4$)Alkoxy, Phenyl oder Benzyl, die beide im Phenylring, vorzugsweise bis zu dreifach, durch ($C_1$-$C_4$)Alkyl, ($C_1$-$C_4$)-Alkoxy, Halogen oder ($C_1$-$C_4$)Alkoxycarbonyl substituiert sein können, substituiert sein kann,
$R_{10}$,$R_{11}$ unabhängig voneinander Wasserstoff, ($C_1$-$C_4$)Alkyl, Phenyl oder Benzyl, die beide jeweils im Phenylring, vorzugsweise bis zu dreifach, substituiert sein können durch Halogen, ($C_1$-$C_4$)Alkyl, ($C_1$-$C_4$)-Alkoxy, $NO_2$, -CN, $CF_3$ oder ($C_1$-$C_4$)Alkoxycarbonyl, oder $R_{10}$ und $R_{11}$ zusammen den Rest

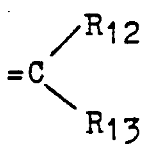 ,

$R_{12}$,$R_{13}$ unabhängig voneinander, ($C_1$-$C_4$)Alkyl, ($C_1$-$C_4$)Alkoxycarbonyl, Phenyl oder Benzyl, die beide im Phenylring, vorzugsweise bis zu dreifach, substituiert sein können durch Halogen, ($C_1$-$C_4$)Alkyl, ($C_1$-$C_4$)-Alkoxy, ($C_1$-$C_4$)Alkoxycarbonyl, $CF_3$, -CN oder $NO_2$,

M 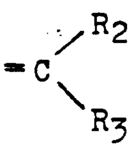 , -S-, 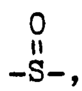 , 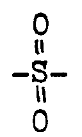 , -O- oder $NR_7$ ;

Q 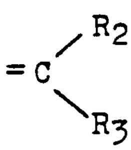 , -S- oder -O-

T 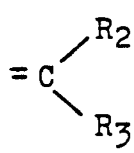 , -S-, 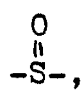 , 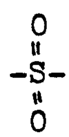 oder -O- ,

X = O oder S
Y = O, S oder NH,
Z = O, S oder $NR_6$,
m = 1, 2, 3 und
n = 1 oder 2 bedeuten,
mit der Maßgabe, daß, wenn Y = O, $R_1$, $R_2$ , $R_3$ = H und Q, T = $CH_2$ bedeuten, M nicht $CH_2$, S, SO oder $SO_2$ sein darf, dadurch gekennzeichnet, daß man

a) für Y = O, S eine Verbindung der Formel II mit einer Verbindung der Formel III,

$Ar-N=C=X$

II

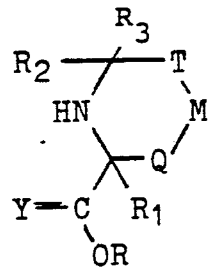

III

worin R = H oder ($C_1$-$C_4$)Alkyl bedeutet, oder

b) eine Verbindung der Formel II mit einem Amin der Formel IV umsetzt

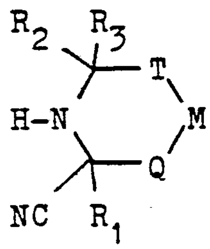

(IV)

oder

c) für Y = O eine unter b) erhaltene Verbindung der Formel I hydrolysiert und die erhaltenen Verbindungen gegebenenfalls in ihre Salze überführt.

2. Verfahren gemäß Anspruch 1, dadurch gekennzeichnet, daß

Ar = Phenyl, das bis zu dreifach substituiert sein kann durch Fluor, Chlor oder Brom, ($C_1$-$C_4$)Alkyl, ($C_1$-$C_4$)-Alkoxy, ($C_3$-$C_4$)Alkenyloxy, ($C_3$-$C_4$)Alkinyloxy, Halo($C_1$-$C_4$)alkyl, Halo($C_1$-$C_4$)alkoxy, -$NHR_1$, ($C_1$-$C_4$)Alkylthio, ($C_1$-$C_4$)Alkylsulfonyl, $NO_2$ oder einen Rest der Formeln

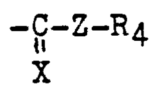

oder

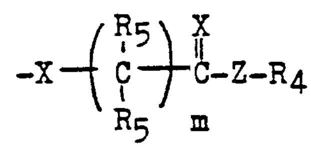

$R_1$ = Wasserstoff, ($C_1$-$C_4$)Alkyl, ($C_1$-$C_4$)Alkoxycarbonyl

$R_2$,$R_3$ = unabhängig voneinander Wasserstoff, ($C_1$-$C_4$)Alkyl, ($C_1$-$C_4$)Alkoxycarbonyl, ($C_1$-$C_4$)Alkoxy($C_1$-$C_4$)-alkoxycarbonyl, Halogen($C_1$-$C_4$)alkoxycarbonyl, ($C_1$-$C_4$)Alkoxycarbonyl($C_1$-$C_4$)alkoxycarbonyl, -CN, Carboxy,

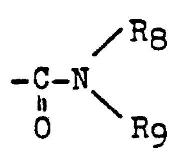

$R_4$ = ($C_1$-$C_4$)Alkyl, Halogen($C_1$-$C_4$)alkyl, ($C_1$-$C_4$)Alkoxyalkyl, ($C_1$-$C_4$)Alkoxycarbonyl($C_1$-$C_4$)alkyl;

$R_5$ = H oder ($C_1$-$C_4$)Alkyl,

$R_7$ = Wasserstoff, ($C_1$-$C_4$)Alkyl,

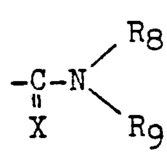

;

Phenyl oder Benzyl, die beide bis zu zweifach durch ($C_1$-$C_4$)Alkyl, ($C_1$-$C_4$)Alkoxy, Halogen, $NO_2$, oder -$CF_3$

substituiert sein können,
$R_8,R_9$ = Wasserstoff, $(C_1\text{-}C_4)$Alkyl, zusammen mit den sie verbindenden N-Atom einem 6-gliedrigen gesättigten Heterocyclus

M = 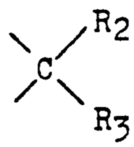 , $-O-$ oder $-NR_7-$

Q = 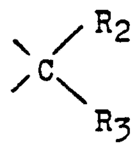

T = 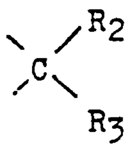 oder $-S-$

X = O oder S,
Y = O oder NH,
Z = O oder S und
m = 1 bedeuten.

Europäisches Patentamt

# EUROPÄISCHER RECHERCHENBERICHT

Nummer der Anmeldung

EP 87118639.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| D,A | EP - A1 - 0 104 532 (NIPPON KAYAKU)<br>* Ansprüche 1,6,7; Zusammenfassung *<br>-- | 1-6 | C 07 D 471/04<br>C 07 D 487/04<br>C 07 D 498/04<br>C 07 D 513/04<br>A 01 N 43/90<br>//(C 07 D 471/04<br>C 07 D 235:00<br>C 07 D 221:00) |
| D,A | US - A - 4 179 276 (CHENG)<br>* Spalte 1, Zeile 45 - Spalte 2, Zeile 50; Spalte 3, Zeilen 20-55 *<br>-- | 1-6 | |
| D,A | EP - A2 - 0 070 389 (SUMITOMO)<br>* Ansprüche 1,9-14 *<br>-- | 1-6 | |
| A | US - A - 4 138 242 (GODDARD)<br>* Ansprüche 1,2; Spalte 2, Zeilen 28-45; Spalte 3, Zeilen 16-45 *<br>-- | 1-6 | **RECHERCHIERTE SACHGEBIETE (Int. Cl. 4)** |
| A | GB - B - 1 503 244 (MITSUBISHI)<br>* Ansprüche 1,22,37,41; Seite 1, Zeilen 6-9 *<br>-- | 1-6 | C 07 D 471/00<br>C 07 D 487/00<br>C 07 D 498/00<br>C 07 D 513/00<br>A 01 N 43/00 |
| A | US - A - 3 958 976 (GODDARD)<br>* Spalte 1, Zeile 53 - Spalte 2, Zeile 15; Spalten 3,4 *<br>-- | 1-6 | |
| A | DE - A - 1 445 797 (FARBWERKE HOECHST)<br>* Seite 1, Formel I; Anspruch 1*<br>---- | 1,3 | |

Der vorliegende Recherchenbericht wurde für alle Patentansprüche erstellt.

| Recherchenort | Abschlußdatum der Recherche | Prüfer |
|---|---|---|
| WIEN | 03-03-1988 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82